# EUROPEAN PATENT APPLICATION

(11) **EP 4 427 787 A1**
(43) Date of publication of application: **11.09.2024**
(21) Application number: 23215479.9
(22) Date of filing: 11.12.2023
(51) Int. Cl.: A61M 25/02, A61F 5/443, A61F 5/453

(54) **ADHESION LOCATION ADJUSTMENT FOR SELF-ADHERING MALE EXTERNAL CATHETERS**

(30) Priority: 12.12.2022 US 202263387070 P
(71) Applicant: Minnesota Medical Technologies Corporation, Stewartville, Minnesota 55976 (US)
(72) Inventor: CONWAY, Anthony J., Stewartville, Minnesota, 55976 (US); CONWAY, Philip J., Stewartville, Minnesota, 55976 (US); GRINDE, Sarah L., Stewartville, Minnesota, 55976 (US)
(74) Representative: Torredimare, Luca

(57) **Abstract**

Devices and/or methods for treating and/or managing urinary incontinence. A self-adhering male external catheter (MEC) is worn by the patient. Adhesive applied to a surface of the MEC adheres the MEC to the patient's skin. An adhesion zone of the MEC or another MEC to the skin is adjusted during the course of the treatment or management to allow the skin to rest and rejuvenate and reduce incidence of skin problems at the site of skin to adhesive contact.

## Description

### Cross-Reference to Related Application

This application claims priority to and the benefit of U.S. Provisional Application No. 63/387,070 filed December 12, 2022, the contents of which are hereby incorporated by reference in their entirety.

### Field of the Invention

The present invention relates to devices and methods for managing urinary incontinence.

### Background of the Invention

Self-adhesive male external catheters (MECs) help individuals manage urinary incontinence successfully. However, with continued use of these products over a period of time, skin irritation and/or maceration can occur at the site of adhesion of the MEC due to the prolonged period with which the adhesive of the MEC is in contact with the skin of the patient, which can inhibit the skin from resting, healing and regenerating. In addition, the act of removing the MEC pulls at the skin at the location of the MEC's adhesive contact with the skin, which can cause, and/or further exacerbate, maceration and irritation of already sensitive skin.

### Summary of the Invention

In general terms, the present disclosure is directed to one or more self-adhesive male external catheters, for which an adhesion location can be adjusted.

In further general terms, the present disclosure is directed to a system for treating or managing urinary incontinence, the system including a plurality of male external catheters with differently configured adhesion zones.

In further general terms, the present disclosure is directed to a method of treating or managing urinary incontinence, wherein an adhesion location, or area, of one or more male external catheters to a patient is adjusted during a course of the treatment or of the management.

In further general terms, the present disclosure is directed to a method of treating or managing urinary incontinence that reduces a likelihood of skin irritation or maceration at the site of a male external catheter.

Advantageously, aspects of the present disclosure, can allow for restful periods during which the skin has the opportunity for less maceration and less adhesive pulls on irritated skin in order to prevent tearing and other damage to the skin.

According to certain aspects, the present disclosure is directed to a method of treating or managing male urinary incontinence, wherein an area of skin of a patient serves as a catheter adhesion location or area during a first time period of the treatment or the management while a male external catheter (MEC) is adhered to the patient, and wherein the area of the skin does not serve as a catheter adhesion location during a second time period of the treatment or the management while the MEC or another MEC is adhered to the patient.

According to certain specific aspects, the present disclosure is directed to a system for treating or managing urinary incontinence, including: a plurality of male external catheters of the same size, the plurality of male external catheters including sheaths with interior surfaces having differently configured adhesion zones of exposed adhesive for adhering, with the adhesive, each of the plurality of male external catheters to a different adhesion location on a patient's skin.

According to further specific aspects, the present disclosure is directed to a device for treating or managing urinary incontinence, including: a male external catheter including a sheath, the sheath including an interior surface and an exterior surface, the interior surface including two zones of exposed adhesive separated by a zone of no exposed adhesive.

According to further specific aspects, the present disclosure is directed to: a method of treating or managing male urinary incontinence, including: (a) adhering a first male external catheter to a first adhesion area of a patient's skin for a first period of time; (b) after the first period of time, removing the first male external catheter from the patient; and (c) after (b) adhering a second male external catheter to a second adhesion area of the patient's skin without adhering with adhesive the second male external catheter to the first adhesion area.

### Brief Description of the Figures

Figure 1 schematically illustrates an example male external catheter according to an example first configuration and in accordance with the present disclosure.
Figure 2 schematically illustrates an example male external catheter according to an example second configuration and in accordance with the present disclosure.
Figure 3 schematically illustrates an example male external catheter according to an example third configuration and in accordance with the present disclosure.
Figure 4 depicts an example method of using male external catheters in accordance with the present disclosure.
Figure 5 schematically illustrates an example system of male external catheters in accordance with the present disclosure.

### Detailed Description of the Invention

The present invention relates to systems, devices and methods for urinary incontinence management and/or treatment and, more particularly, for male urinary incontinence management and/or treatment. As used herein, "management" can also refer to treatment, and "treatment" can also refer to management.

There are several drawbacks to the use of male external catheters. For example, the act of removing the MEC pulls at the skin at the location of the MEC's adhesive contact with the skin, which can cause, and/or further exacerbate, maceration and/or irritation of already sensitive skin.

Typically, a MEC is replaced with a fresh MEC at regular or irregular intervals, e.g., after a number of hours or a number of days. Because the adhesive configuration of the fresh MEC that is worn by the patient is the same as the adhesive configuration of the previous MEC worn by the patient, the same area of skin of the patient remains in contact with adhesive, prolonging that area of skin's duration of adhesive contact.

There is a tendency for skin issues to develop at the site of prolonged and/or repeated adhesive contact between the patient's skin and the male external catheter (MEC). For instance, prolonged contact with adhesive of a MEC can cause skin irritation and/or skin maceration. When the MEC is removed (e.g., to replace the MEC with a fresh one), there is a tendency for the skin to be pulled with the adhesive. This skin, softened by the adhesive, tends to tear at the site of the adhesive, which can cause pain, and can also cause a sore and/or infection to develop. To mitigate pain or treat an infection, patients whose skin has been negatively impacted in one or more of these ways by adhesive contact of the MEC may opt to stop using a MEC entirely or for a period of time while the skin heals, which can be detrimental to the patients' urinary incontinence management, as well as to their overall health.

The systems, devices and methods of the present disclosure aspects can advantageously allow for restful periods during which the skin has the opportunity for less maceration and less adhesive pulls on irritated skin in order to prevent tearing and other damage to the skin.

The systems, devices and methods of the present disclosure can provide improvements to urinary incontinence management and/or treatment by reducing the amount of time a MEC is adhered with adhesive to a given area of the patient's skin. Because adhesive to skin contact time for a given area of skin is reduced, iatrogenic symptoms and other negative consequences caused by wearing of the MEC, such as those described above, can be mitigated. For example, a sore or broken skin that results from adhesive pulling the skin during removal of a first MEC will not be exposed to adhesive when a subsequent MEC having a different adhesive configuration is worn, thereby allowing the sore or broken skin time to heal. Similarly, in accordance with the present disclosure, if a patient before or during MEC use has an area of impaired skin (e.g., irritation, a wound, or any skin impairment caused by any skin condition), a MEC having an adhesive configuration that will avoid contact with the impaired skin area can be advantageously selected to be worn.

As used herein, a male external catheter (MEC) is an external catheter that is worn by a patient on an exterior of the skin of the patient's penis. An equivalent term for a MEC, as used herein, is a condom catheter.

A typical MEC is a device that includes a cylindrical sheath that receives the patient's penis. An interior surface of the sheath includes adhesive that adheres the sheath to the skin of the penis. As used herein, "adhesive" refers to any medical grade adhesive known now or in the future that is suitable for adhering the sheath of the MEC to the patient's skin.

At a distal end of the sheath there is an interface. The interface defines a port through which urine can flow. In addition, the interface is configured to couple to a receptacle (e.g., directly, or via a tube) that can collect expelled urine. The receptacle can be, e.g., a urine collection bag. In some examples, the interface can include a one-way valve or nozzle that can inhibit or prevent urine from flowing back into the sheath once it has passed through the port. Towards the distal end of the sheath, the sheath can narrow, forming a funnel to direct urine through the port and into the receptacle.

In some examples, a proximal portion of the MEC sheath can be rolled up before use (similar to a prophylactic condom), which can facilitate sheathing of the sheath onto the penis by unrolling or unfurling the proximal portion and thereby advancing the sheath onto the penis. Unused MECs can be stored and shipped in a box or other package, for example, in their rolled up configurations. To use the MEC, the sheath is unrolled or unfurled, such that the adhesive contacts the skin to hold the sheath in place.

Throughout the several views, like numbers refer to like parts.

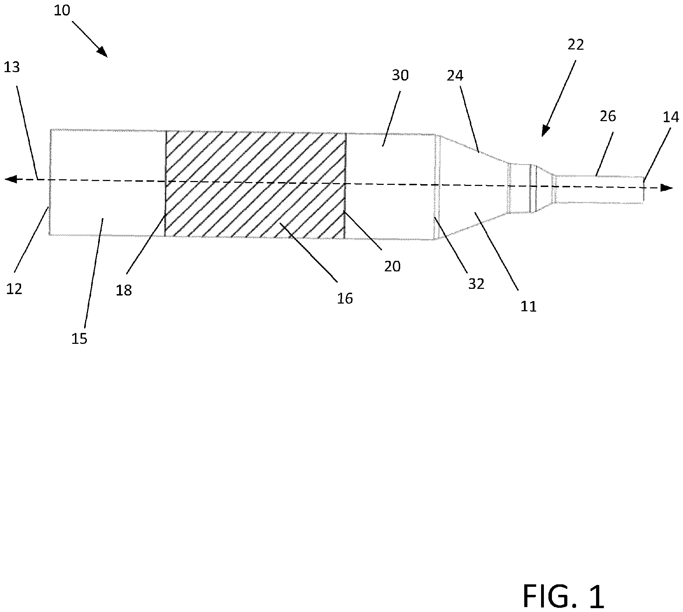

Figure 1 schematically illustrates an example male external catheter 10 according to an example first configuration and in accordance with the present disclosure. The MEC 10 is depicted with its sheath unfurled. The MEC 10 includes a body 11. The body 11 defines a longitudinal axis 13 and extends along the axis 13 from a proximal end 12 of the body 11 to a distal end 14 of the body 11.

The MECs of the present disclosure can be constructed of flexible, medical grade material that can be in contact with human skin for extended periods of time, that resists water penetration into the material, and to which adhesive can be applied. In some examples, the MECs are constructed of silicone. The silicone can be, but need not be, transparent. In some examples, multiple features, or all features, of the MEC are of unitary construction, e.g., formed in a single molding process. In other examples, features can be molded separately and then fused together.

The body 11 includes a sheath 15 extending to the proximal end 12 of the body 11. The sheath 15 can be hollow and cylindrical (or approximately cylindrical), including an interior surface and an exterior surface. The sheath 15 is configured to receive a patient's penis. The body 11 includes a funnel 24 extending from a distal end of the sheath 15 and narrowing towards the axis 13 as it extends distally from the sheath 15. The funnel 24 can be a hollow frusto-cone, and includes an interior surface and an exterior surface. Extending distally from the funnel 24 is an interface 22. The interface 22 can include a port through which urine can flow, and a coupling element 26 (e.g., a tube) configured to couple the MEC 10 to a receptacle (not shown) that can collect expelled urine. The receptacle can be, e.g., a urine collection bag. In some examples, the interface 22 can include a one-way valve or nozzle that can inhibit or prevent urine from flowing back into the sheath 15 once it has passed through the port. The funnel 24 can help to direct urine flow through the interface 22 and into the receptacle for collection.

The interior surface of the sheath 15 includes a zone 16 (also referred to herein as an adhesion zone) with exposed adhesive that adheres the sheath to the skin of the patient. The zone 16 extends from a proximal end 18 of the zone 16 to a distal end 20 of the zone 16. The zone can be cylindrical between the ends 18 and 20. That is, the entire interior surface of the sheath between the ends 18 and 20 can have exposed adhesive thereon.

Though not in the depicted example, the end 18 can, in some examples, coincide with the proximal end of the sheath.

The interior surface of the sheath 15 includes a zone 30 that does not include exposed adhesive. In some examples, the zone 30 can include adhesive that is covered up and therefore not exposed. For instance, a layer of silicone or another material can be applied over adhesive in the zone 30. In other examples, no adhesive is applied in the zone 30. In some examples the zone 30 can include a non-adhesive therapeutic substance (e.g., a vitamin-rich or steroidal ointment) to enhance healing of the skin that is in contact with the zone 30. The zone 30 extends from a proximal end 20 of the zone 16 to distal end 32 of the zone 30. In this example, the distal end 32 of the zone 30 coincides with the distal end of the sheath 15.

The positioning and sizes of the zones 16 and 30 make up an adhesive configuration for the MEC 10.

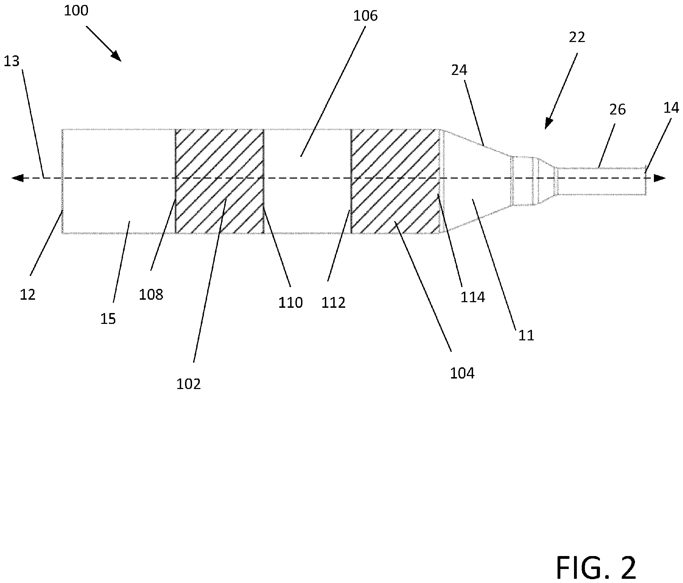

Figure 2 schematically illustrates an example male external catheter 100 according to an example second configuration and in accordance with the present disclosure.

With the exception of its adhesive configuration, the MEC 100 is otherwise of identical construction to that of the MEC 10. In the interest of brevity, discussion of Figure 2 will focus largely on the differences between the MEC 100 and the MEC 10.

The interior surface of the sheath 15 of the body 11 of the MEC 100 includes a first zone 102 (also referred to as an adhesion zone) with exposed adhesive that adheres the sheath to the skin of the patient. The first zone 102 extends from a proximal end 108 of the zone 102 to a distal end 110 of the zone 102. The zone 102 can be cylindrical between the ends 108 and 110. That is, the entire interior surface of the sheath between the ends 108 and 110 can have exposed adhesive thereon.

The interior surface of the sheath 15 of the body 11 of the MEC 100 also includes a second zone 104 (also referred to as an adhesion zone) with exposed adhesive that adheres the sheath to the skin of the patient. The second zone 104 extends from a proximal end 112 of the zone 104 to a distal end 114 of the zone 104. The zone 104 can be cylindrical between the ends 112 and 114. That is, the entire interior surface of the sheath between the ends 112 and 114 can have exposed adhesive thereon.

Between the zones 102 and 104, and juxtaposed to the zones 102 and 104, the interior surface of the sheath 15 of the body 11 of the MEC 100 includes a zone 106 that does not include exposed adhesive. In some examples, the zone 106 can include adhesive that is covered up and therefore not exposed. For instance, a layer of silicone or another material can be applied over adhesive in the zone 106. In other examples, no adhesive is applied in the zone 106. In some examples the zone 106 can include a non-adhesive therapeutic substance (e.g., a vitamin-rich or steroidal ointment) to enhance healing of the skin that is in contact with the zone 106. The zone 106 extends from a proximal end 110 of the zone 106 to a distal end 112 of the zone 106. That is, the proximal end of the zone 106 coincides with the distal end of the zone 102, and the distal end of the zone 106 coincides with the proximal end of the zone 104. The zone 106 thus separates the zones 102 and 104 from each other.

In the MEC 10 of Figure 1, the region of the interior surface of the sheath corresponding to the zone 106 of the MEC 100 of Figure 2 includes exposed adhesive. In the MEC 100 of Figure 2, the region of the interior surface corresponding to the zone 30 of the MEC 10 of Figure 1 includes exposed adhesive. Thus, as between the MEC 10 and the MEC 100, the exposed adhesive is staggered. During urinary incontinence treatment or management, the patient can replace the MEC 10 with the MEC 100 (or vice versa) after a period of time, advantageously allowing an area of the patient's skin time to rest and/or heal from adhesive application and skin-pulling adhesive removal from a previously worn MEC. For example, an area of skin of the patient corresponding to the zone 106 rests from adhesive contact while the patient wears the MEC 100, whereas that area of skin is in contact with adhesive when the patient wears the MEC 10. Similarly, an area of skin corresponding to the zone 30 rests from adhesive contact while the patient wears the MEC 10, whereas that area of skin is in contact with adhesive when the patient wears the MEC 100.

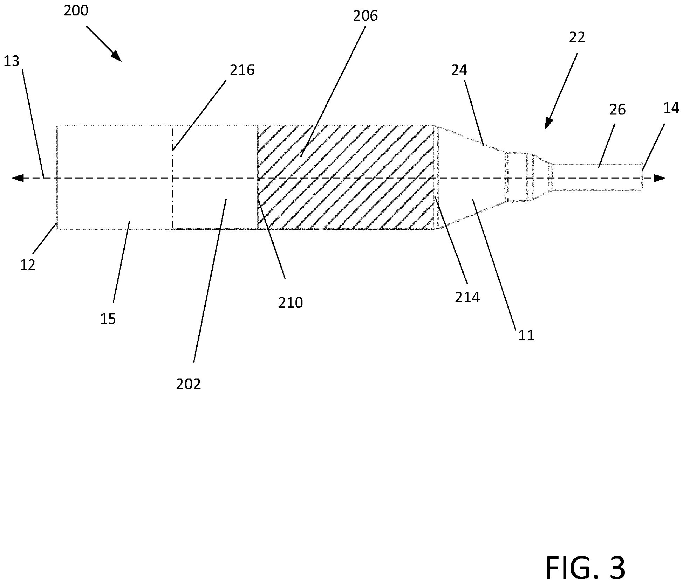

Figure 3 schematically illustrates an example male external catheter 200 according to an example third configuration and in accordance with the present disclosure.

With the exception of its adhesive configuration, the MEC 200 is otherwise of identical construction to that of the MEC 10 and the MEC 100. In the interest of brevity, discussion of Figure 3 will focus largely on the differences between the MEC 200, and each of the MEC 10 and the MEC 100.

The interior surface of the sheath 15 of the body 11 of the MEC 200 includes a zone 206 (also referred to as an adhesion zone) with exposed adhesive that adheres the sheath to the skin of the patient. The zone 206 extends from a proximal end 210 of the zone 206 to a distal end 214 of the zone 102, which coincides with the distal end of the sheath 15. The zone 206 can be cylindrical between the ends 210 and 214. That is, the entire interior surface of the sheath between the ends 210 and 214 can have exposed adhesive thereon.

The interior surface of the sheath 15 of the body 11 of the MEC 200 includes a zone 202 that does not include exposed adhesive. In some examples, the zone 202 can include adhesive that is covered up and therefore not exposed. For instance, a layer of silicone or another material can be applied over adhesive in the zone 202. In other examples, no adhesive is applied in the zone 202. In some examples the zone 202 can include a non-adhesive therapeutic substance (e.g., a vitamin-rich or steroidal ointment) to enhance healing of the skin that is in contact with the zone 202. The zone 202 extends from a proximal end 216 (illustrated in phantom) of the zone 202 to a distal end 210 of the zone 206, which coincides with the proximal end of the zone 206.

In the MEC 10 of Figure 1 and the MEC 100 of Figure 2, the region of the interior surface of the sheath corresponding to the zone 202 of the MEC 200 of Figure 3 includes exposed adhesive. In the MEC 200 of Figure 3, the region of the interior surface corresponding to the zone 30 of the MEC 10 of Figure 1, and the region of the interior surface corresponding to the zone 106 of the MEC 100 of Figure 2 include exposed adhesive. Thus, as between the MEC 10 and the MEC 200, the exposed adhesive is staggered, and as between the MEC 100 and the MEC 200, the exposed adhesive is staggered. During urinary incontinence treatment or management, the patient can replace the MEC 10 or the MEC 100 with the MEC 200 (or vice versa) after a period of time, advantageously allowing an additional area of the patient's skin time to rest and heal from adhesive application and skin-pulling adhesive removal from the previously removed MEC. For example, an area of skin of the patient corresponding to the zone 202 rests from adhesive contact while the patient wears the MEC 200, whereas that area of skin is in contact with adhesive when the patient wears the MEC 10 and the MEC 200.

Though not in the depicted example, the end 216 can, in some examples, coincide with the proximal end of the sheath.

As among the MECs 10, 100 and 200, the overall adhesion region can be considered divided into thirds, or approximately thirds, with the adhesion zone of each MEC overlapping with half, or approximately half, of the adhesion zone of each other MEC. A treatment or management plan that uses all three of the MECs 10, 100 and 200, can be divided into three time periods, e.g., of approximately equal length. The patient wears one of the MECs 10, 100 and 200 for the first time period, then replaces the MEC with another of the MECs 10, 100, 200 for the second time period, and then replaces the MEC again with the last of the MECs 10, 100, 200 for the third time period. For example, if a given treatment or management period is three weeks, the patient can wear the MEC 10 for one week, then replace the MEC 10 with the MEC 100 for one week, and then replace the MEC 100 with the MEC 200 for the third week. Thus, during each week, a different third of the skin area corresponding to the overall adhesive configuration of the MECs 10, 100, 200 is advantageously and therapeutically able to rest without adhesive contact.

Any order of usage among the MECs 10, 100, and 200 can be employed. For example, a patient can wear the MEC 10, then the MEC 200, then the MEC 100, or the MEC 100, then the MEC 10, then the MEC 200, or the MEC 100 then the MEC 200, then the MEC 10, or the MEC 200, then the MEC 100, then the MEC 10, or the MEC 200, then the MEC 10, then the MEC 100. Generally, the number of orders of MECs that can be worn by the patient is n!, wherein n is a positive integer greater than 1 corresponding to the number of different adhesive configurations among the group of MECs.

By way of another example, if n is 2, then a system of MECs for treatment or management of male urinary incontinence includes MECs of two different, and staggered adhesive configurations, and a typical treatment or management period can be divided into two subperiods, one during which the patient wears one of the MECs, and another during which the patient wears the other of the MECs. In this example, the overall adhesion region can be considered divided into halves, or approximately halves, with the adhesion zone of each MEC not overlapping with the adhesion zone of the other MEC. Thus, during each treatment or management period (or interval), a different half of the skin area corresponding to the overall adhesive configuration of the two differently configured MECs is advantageously and therapeutically able to rest and heal from adhesive contact and skin pulling adhesive removal.

By way of another example, if n is 4, then a system of MECs for treatment or management of male urinary incontinence includes MECs of four different, and staggered adhesive configurations, and a typical treatment or management period can be divided into four subperiods, during each of which the patient wears a different one of the four MECs, Thus, during each treatment or management period (or interval), a different quarter of the skin area corresponding to the overall adhesive configuration of the four differently configured MECs is advantageously and therapeutically able to rest without adhesive contact.

In other examples, the number n of different adhesive configurations can be more than four and the treatment or management plan can be adjusted accordingly.

Other possibilities for shifting or staggering areas of adhesive contact of the patient's skin over a urinary incontinence treatment or management period by providing MECs with differently configured adhesion zones are possible.

In some examples, a total length of the exposed adhesive along the axis 13, regardless of the number of zones of the exposed adhesive, is equal for all of the MECs 10, 100 and 200. The total length of the exposed adhesive for all MECs can be selected to provide at least enough adhesive to effectively secure each MEC to the patient for the desired time period of treatment or management of urinary incontinence.

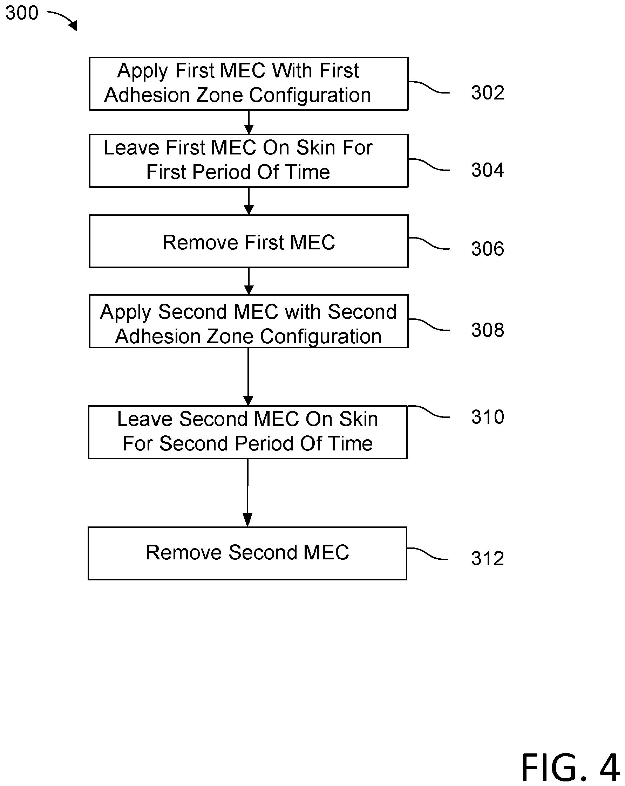

Figure 4 depicts an example method 300 of using male external catheters in accordance with the present disclosure. For example, the method 300 can be used with at least any two of the MECs 10, 100 and 200. In some examples, the method 300 is performed by a patient. In some examples, the method 300 is performed by a medical provider. In some examples, the method 300 can be performed by both the patient and a medical provider.

At a step 302 of the method 300, a first MEC with a first adhesion zone configuration is applied to the patient's skin. In some examples, if the patient already has an area of skin impairment, the initial MEC is chosen with an adhesion zone configuration that will avoid adhesive contact with the area of skin impairment, to allow that area time to heal.

At a step 304 of the method 300, the first MEC is left on the skin for a first period of time.

At a step 306, the first MEC is removed from the patient.

At a step 308, a second MEC with a second adhesion zone configuration that is different from the first adhesion zone configuration is applied to the patient's skin.

At a step 310, the second MEC is left on the skin for a second period of time, that can be equal to, longer than, or shorter than the first period of time.

At a step 312, the second MEC is removed from the patient.

In some examples of the method 300, one or more additional MECs with adhesive configurations different from those of the first and second adhesive configurations can be applied for additional periods of time.

In some examples of the method 300, the steps 302 through 312 can be repeated one or more times, using an additional set (or sets) of the MECs having the first adhesion zone configuration and the second adhesion zone configuration.

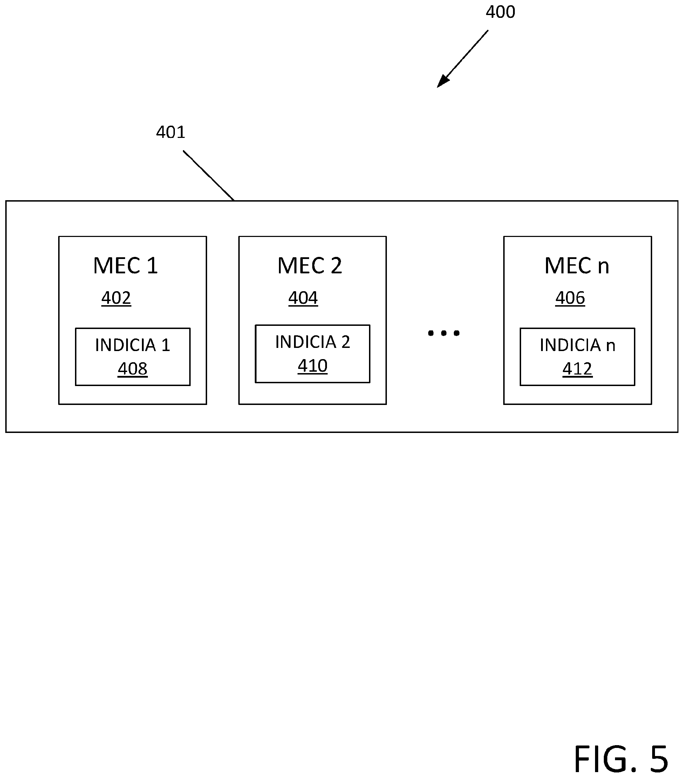

Figure 5 schematically illustrates an example system 400 of male external catheters in accordance with the present disclosure. The system 400 can be self-administered by the patient, or administered to the patient with the help of a medical provider.

The system 400 includes a container (such as a package) 401. In some examples, the container can correspond to multiple containers, for example, if each MEC is individually packaged. The container 401 includes multiple MECs. At least two of the MECs contained in the package 401 have differently configured adhesive configurations. In the example shown, the package 401 includes at least MEC 1 402, MEC 2 404, and up to MEC n 406, where n is a positive integer that is, in this example greater than 2. In other examples, n can be 2. In one example instance of the system 400, n is 3, and MECs 402, 404 and 406 correspond, respectively, to the MECs 10, 100 and 200 of Figures 1, 2 and 3 described above.

Each MEC in the package 401 includes indicia. MEC 1 402 includes indicia 408. MEC 2 404 includes indicia 410. MEC 3 406 includes indicia 412. The indicia 408, 410, 412 can indicate the adhesive configuration of the corresponding or associated MEC, and/or simply indicate that the adhesive configuration of that MEC is different from the adhesive configuration of a MEC with different indicia.

The indicia 408, 410 and 412 can include any suitable indicators. For example, the indicia can include colors, with different colors corresponding to different adhesive configurations. In another example, the indicia can include text that, e.g., describes the adhesive configuration and/or the treatment or management period for which that the MEC should be worn. In another example, the indicia can include an image or diagram that illustrates the adhesive configuration of that MEC. In some examples, the indicia 408, 410 and 412 can include combinations of one or more different types of indicators, such as combinations of both colors and diagrams or combinations of text, colors and diagrams.

The indicia 408, 410, 412 can facilitate selection by the patient or medical provider of the appropriate MEC for a given time period of treatment or management for male urinary incontinence.

As used herein, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the term "configured" describes a system, apparatus, or other structure that is constructed or configured to perform a particular task or adopt a particular configuration. The term "configured" can be used interchangeably with other similar phrases such as arranged and configured, constructed and arranged, adapted and configured, adapted, constructed, manufactured and arranged, and the like.

The invention has been described with reference to various specific and preferred embodiments and techniques. However, it should be understood that many variations and modifications may be made while remaining within the spirit and scope of the invention.

## Claims

1. A system for treating or managing urinary incontinence, comprising:
a plurality of male external catheters of the same size, the plurality of male external catheters including sheaths with interior surfaces having differently configured adhesion zones of exposed adhesive for adhering, with the adhesive, each of the plurality of male external catheters to a different adhesion location on a patient's skin.

2. A method of using the system of claim 1, comprising:
(a) positioning a first of the plurality of male external catheters on the patient's skin, including adhering the adhesion zone of the first of the plurality of male catheters to a first adhesion area on the patient's skin for a first period of time;
(b) removing, after the first period of time, the first of the plurality of male external catheters from the patient; and
(c) positioning a second of the plurality of male external catheters on the patient's skin, including adhering the adhesion zone of the second of the plurality of male catheters to a second adhesion area on the patient's skin for a second period of time, without adhering with adhesive the second of the plurality of male catheters to the first adhesion area.

3. The system of any of claims 1-2, wherein each of the male external catheters is associated with indicia indicating a configuration of the adhesion zone of the associated male external catheter.

4. The system of any of claims 1-3, wherein the indicia include different colors.

5. The system of any of claims 1-4, wherein the indicia include diagrams.

6. The system of any of claims 1-5, wherein the indicia include text.

7. The system of any of claims 1-6, including exactly two of the external male catheters.

8. The system of claim 7, wherein the adhesion zone of one of the external male catheters does not overlap with the adhesion zone of the other of the external male catheters.

9. The system of any of claims 1-6, including exactly three of the external male catheters.

10. The system of claim 9, wherein the adhesion zone of one of the external male catheters overlaps with approximately one half of the adhesion zone of each of the others of the external male catheters.

11. A device for treating or managing urinary incontinence, comprising:
a male external catheter including a sheath, the sheath including an interior surface and an exterior surface, the interior surface including two zones of exposed adhesive separated by a zone of no exposed adhesive.

12. The device of claim 11, wherein the zone of no exposed adhesive includes adhesive that is covered.

13. The device of claim 12, wherein the covered adhesive is covered with a layer of silicone.

14. The device of claim 11, wherein the zone of no exposed adhesive includes no adhesive.

15. The device of any of claims 11-14,
wherein the sheath is cylindrical; and
wherein the device further comprises a funnel and an interface, the interface being configured to couple the device to a urine receptacle.

16. A method of treating or managing male urinary incontinence, comprising:
(a) adhering a first male external catheter to a first adhesion area of a patient's skin for a first period of time;
(b) after the first period of time, removing the first male external catheter from the patient; and
(c) after (b) adhering a second male external catheter to a second adhesion area of the patient's skin without adhering with adhesive the second male external catheter to the first adhesion area.

17. The method of claim 16, further comprising:
(d) after (c) removing the second male external catheter from the patient after a second period of time; and
(e) after (d), adhering a third male external catheter to the patient.

18. The method of claim 17, wherein the second period of time is equal to the first period of time.

19. The method of any of claims 16-18, further comprising:
(f) after (e), removing the third male external catheter from the patient after a third period of time.

20. The method of claim 19, wherein the third period of time is equal to the first period of time.

21. The method of any of claims 16-20, wherein the third male external catheter is adhered to a third adhesion area of the patient's skin without adhering with adhesive the third male external catheter to at least a portion of the first adhesion area and to at least a portion of the second adhesion area.

22. The method of any of claims 16-20, wherein the third male external catheter is adhered to a third adhesion area of the patient's skin, the third adhesion area being the same as the first adhesion area.

23. The method of any of claims 16-22, wherein at least one of the first male catheter or the second male catheter is selected based on an adhesive configuration of the first male catheter or the second male catheter and further based on a location of an impaired area of the patient's skin, such that no adhesive of the adhesive configuration is adhered to the impaired area of the patient's skin.
